# EUROPÄISCHE PATENTSCHRIFT

(11) **EP 1 868 727 B1**
(45) Veröffentlichungstag und Bekanntmachung des Hinweises auf die Patenterteilung: **05.07.2017**
(21) Anmeldenummer: 06724046.5
(22) Anmeldetag: 05.04.2006
(51) Int. Cl.: B02C 19/00, A61F 2/46

(54) **KNOCHENMÜHLE**
BONE MILL
MOULIN A OS

(30) Priorität: 06.04.2005 DE 102005017001
(43) Veröffentlichungstag der Anmeldung: 26.12.2007
(73) Patentinhaber: Aesculap AG, 78532 Tuttlingen (DE)
(72) Erfinder: MORALES, Pedro, 78532 Tuttlingen (DE); BECK, Thomas, 78591 Durchhausen (DE); REBHOLZ, Werner, 78579 Neuhausen (DE); STRICKER, Andres, 78464 Konstanz (DE)
(74) Vertreter: Hoeger, Stellrecht & Partner Patentanwälte mbB
(86) Internationale Anmeldenummer: PCT/EP2006/003098
(87) Internationale Veröffentlichungsnummer: WO 2006/105950

(56) Entgegenhaltungen:
- EP-A- 0 590 219
- EP-A- 0 839 500
- DE-A1- 19 846 702
- US-A- 5 918 821
- US-A1- 2002 070 299
- US-B1- 6 287 312

## Beschreibung

Die vorliegende Erfindung betrifft eine Knochenmühle zum Zerkleinern eines Mahlguts in Form von Knochen oder Knochenmaterial, mit einem Aufnahmeraum für das Mahlgut, einer Zerkleinerungsvorrichtung und einem Vorschubelement zum Bewegen des Mahlguts im Aufnahmeraum in Richtung auf die Zerkleinerungsvorrichtung hin, wobei der Aufnahmeraum eine Austrittsöffnung aufweist, die mindestens teilweise von der Zerkleinerungsvorrichtung bedeckt ist, wobei der Aufnahmeraum relativ zur Zerkleinerungsvorrichtung bewegbar ist und wobei der Aufnahmeraum relativ zur Zerkleinerungsvorrichtung um eine Drehachse verdrehbar ist.

Knochenmühlen der eingangs beschriebenen Art werden in der Chirurgie eingesetzt, insbesondere auch im Bereich der Mund-, Kiefer- und Gesichtschirurgie, um Knochen oder Knochenmaterial auf eine definierte maximale Korngröße zu zerkleinern.

Eine Knochenmühle der eingangs beschriebenen Art ist beispielsweise aus der DE 198 46 702 A1 bekannt. Weitere Knochenmühlen sind in der EP 0839 500 A1, der US 2002/0070299 A1, der EP 0590 219 A1, der US 6,287,312 B1 sowie der US 5,918,821 offenbart.

Es ist Aufgabe der vorliegenden Erfindung, eine Knochenmühle der eingangs beschriebenen Art so zu verbessern, dass das Mahlgut auf einfache und schonende Weise zerkleinert werden kann.

Diese Aufgabe wird bei einer Knochenmühle der eingangs beschriebenen Art erfindungsgemäß dadurch gelöst, dass der Aufnahmeraum einen bezogen auf die Drehachse konvex gekrümmten Innenwandabschnitt aufweist.

Mit einer solchen Knochenmühle kann Knochen oder Knochenmaterial besonders schonend und nahezu rückstandsfrei zerkleinert werden. Mit dem Vorschubelement kann eine geringe Druckkraft auf das Mahlgut ausgeübt werden, welches aufgrund der Relativbewegung des Aufnahmeraums zur Zerkleinerungsvorrichtung über diese bewegt und dadurch zerkleinert werden kann. Damit die Drehachse außerhalb des Aufnahmeraums verlaufen kann, ist es günstig, wenn der Aufnahmeraum einen bezogen auf die Drehachse konvex gekrümmten Innenwandabschnitt aufweist.

Vorteilhaft ist es, wenn der Aufnahmeraum eine Einfüllöffnung zum Einfüllen des Mahlguts aufweist und wenn die Einfüllöffnung mit dem Vorschubelement teilweise oder vollständig verschließbar ist. Dadurch wird erreicht, dass das in den Aufnahmeraum eingefüllte Mahlgut nicht wieder durch die Einfüllöffnung austreten kann.

Um sicherzustellen, dass das Mahlgut nach dem Einfüllen in dem Aufnahmeraum verbleibt und insbesondere vollständig mit dem Vorschubelement gegen die Zerkleinerungsvorrichtung bewegt werden kann, ist es günstig, wenn das Vorschubelement formschlüssig in den Aufnahmeraum einführbar ist. Insbesondere können der Aufnahmeraum und das Vorschubelement so ausgebildet sein, dass das Vorschubelement in einer maximal eingeführten Stellung den Aufnahmeraum vollständig ausfüllt. Alternativ wäre es auch denkbar, das Vorschubelement so auszubilden, dass zumindest ein Querschnitt des Aufnahmeraums in jeder Stellung nach Einführen des Vorschubelements in den Aufnahmeraum vom Vorschubelement vollständig verschlossen wird.

Für eine besonders effiziente Zerkleinerung des Mahlguts ist es vorteilhaft, wenn die Zerkleinerungsvorrichtung die gesamte Austrittsöffnung bedeckt. Insbesondere dann, wenn die Zerkleinerungsvorrichtung derart ausgebildet ist, dass das zu zerkleinernde Mahlgut beim Zerkleinern durch diese hindurchtritt, kann so die gesamte Austrittsöffnung zum Zerkleinern ausgenutzt werden. Besonders einfach wird der Aufbau der Knochenmühle, wenn der Aufnahmeraum relativ zur Zerkleinerungsvorrichtung um eine Drehachse verdrehbar ist. Der beispielsweise durch eine Mahlkammer gebildete Aufnahmeraum ermöglicht es, das Mahlgut beispielsweise quer zur Drehachse über die Zerkleinerungsvorrichtung zu bewegen und durch gleichzeitige Einwirkung einer geringen Druckkraft auf das Mahlgut mit der Zerkleinerungsvorrichtung zu zerkleinern.

Günstig ist es, wenn die Austrittsöffnung eine Austrittsebene definiert und die Drehachse die Austrittsebene senkrecht oder im wesentlichen senkrecht schneidet. Dies erlaubt es, infolge der Drehbewegung des Aufnahmeraums und unter gleichzeitiger Einwirkung einer geringen Druckkraft das Mahlgut im Aufnahmeraum quer zur Drehachse über die Zerkleinerungsvorrichtung zu bewegen.

Vorzugsweise schneidet die Drehachse die Austrittsöffnung nicht. Durch diese Ausgestaltung wird erreicht, dass das gesamte Mahlgut mit einer bestimmten Geschwindigkeit relativ zur Zerkleinerungsvorrichtung bewegt wird. Es gibt so keinen Punkt, an dem das Mahlgut relativ zur Zerkleinerungsvorrichtung ruht, was insbesondere der Fall wäre, wenn die Drehachse die Austrittsöffnung schneiden würde, wobei zwar der Aufnahmeraum und dessen Austrittsöffnung zwar relativ zur Zerkleinerungsvorrichtung auch bewegt wird, sich jedoch an einem Punkt mit Nullreibung, nämlich am Schnittpunkt zwischen der Drehachse und der Austrittsöffnung, das Mahlgut ansammeln kann.

Um eine Nullreibung zwischen dem Mahlgut und der Zerkleinerungsvorrichtung zu verhindern, ist vorteilhafterweise vorgesehen, dass die Drehachse außerhalb des Aufnahmeraums verläuft. Beispielsweise kann der Aufnahmeraum in Form einer Kammer ausgebildet sein, die um die Drehachse herum angeordnet ist, beispielsweise konzentrisch zur Drehachse, wodurch sichergestellt wird, dass die Drehachse außerhalb des Aufnahmeraums verläuft.

Vorteilhaft ist es, wenn der Aufnahmeraum einen bezogen auf die Drehachse konkav gekrümmten Innenwandabschnitt aufweist. Auf diese Weise kann eine Querschnittsfläche und damit ein Volumen des Aufnahmeraums maximiert werden.

Um nur eine möglichst dünne Wandstärke zur Ausbildung des Aufnahmeraums vorsehen zu müssen, ist es vorteilhaft, wenn der konkav gekrümmte Innenwandabschnitt konzentrisch um die Drehachse herum angeordnet ist. Beispielsweise kann so der Aufnahmeraum aus einem zylindrischen, die Kammer relativ zu einem Gehäuse rotierbar gelagerten Element geformt werden.

Vorteilhafterweise ist der konvex gekrümmte Innenwandabschnitt konzentrisch oder exzentrisch um die Drehachse herum angeordnet. Bei der vorzugsweise exzentrischen Anordnung des konvex gekrümmten Innenwandabschnitts ist es möglich, das Mahlgut mit einer stets gleichbleibenden Geschwindigkeit relativ zur Zerkleinerungsvorrichtung zu bewegen und zudem eine Nullreibung im Schnittpunkt der Drehachse mit der Zerkleinerungsvorrichtung zu vermeiden.

Ein besonders einfacher Aufbau der Knochenmühle ergibt sich, wenn der Aufnahmeraum mindestens einen parallelen Wandabschnitt aufweist, der parallel zu einer die Drehachse enthaltenden Radialebene verläuft. Außerdem kann so eine gleichbleibende oder zumindest nahezu gleichbleibende Geschwindigkeit des Mahlguts relativ zur Zerkleinerungsvorrichtung erreicht werden.

Weiter vereinfacht sich der Aufbau der Knochenmühle, wenn der mindestens eine parallele Wandabschnitt den mindestens einen konkav gekrümmten Wandabschnitt mit dem mindestens einen konvex gekrümmten Wandabschnitt verbindet.

Vorzugsweise entspricht ein Abstand des parallelen Wandschnitts von der Radialebene etwa dem 0,2-fachen bis 0,4-fachen eines Radius des konkav gekrümmten Innenwandabschnitts. Durch den gewählten Abstand kann eine Geschwindigkeitsvariation des Mahlguts im Aufnahmeraum relativ zur Zerkleinerungsvorrichtung gezielt vorgegeben werden.

Gemäß einer bevorzugten Ausführungsform der Erfindung kann vorgesehen sein, dass eine Zerkleinerungsvorrichtungsaufnahme vorgesehen ist und dass die Zerkleinerungsvorrichtung in die Zerkleinerungsvorrichtungsaufnahme quer zur Drehachse einführbar ist. Diese Ausgestaltung erlaubt es, die Zerkleinerungsvorrichtung, insbesondere zu Reinigungs- oder Wartungszwecken, zu entfernen. Ferner kann die Zerkleinerungsvorrichtungsaufnahme derart ausgebildet sein, dass das relativ zur Zerkleinerungsvorrichtung bewegte Mahlgut keine Drehung der Zerkleinerungsvorrichtung bewirken kann.

Um ein unbeabsichtigtes Lösen oder Entfernen der Zerkleinerungsvorrichtung von oder aus der Zerkleinerungsvorrichtungsaufnahme zu vermeiden, ist es günstig, wenn ein Sicherungselement zum Sichern der Zerkleinerungsvorrichtung in der Zerkleinerungsvorrichtungsaufnahme vorgesehen ist.

Besonders einfach wird der Aufbau der Knochenmühle, wenn das Sicherungselement ein Kugeldruckstück ist. Insbesondere kann bei einer federnd vorgespannten Kugel des Kugeldruckstücks die Federkraft so gewählt werden, dass diese stets größer ist als eine aufgrund einer Bewegung des Mahlguts relativ zur Zerkleinerungsvorrichtung auf die Zerkleinerungsvorrichtung wirkende Kraft. Ein Ein- und Ausführen der Zerkleinerungsvorrichtung in die Zerkleinerungsvorrichtungsaufnahme ist mit einem Kugeldruckstück möglich, ohne dass zusätzliche Teile von einer Bedienperson explizit bewegt werden müßten.

Grundsätzlich wäre es denkbar, die Zerkleinerungsvorrichtung in Form von senkrecht zueinander angeordneten Schneiden auszubilden. Eine definierte Korngröße des Mahlguts lässt sich jedoch auch mit einer Zerkleinerungsvorrichtung erreichen, die eine mit Zähnen versehene Reibe ist. Insbesondere kann die Reibe Durchtrittsöffnungen aufweisen, sodass das zerkleinerte Mahlgut durch diese hindurchtreten kann, wobei jedoch nur zerkleinertes Mahlgut gewünschter Korngröße durch die Durchtrittsöffnungen hindurchtreten kann.

Um das Mahlgut reproduzierbar auf eine definierte Korngröße zu zerkleinern, ist es günstig, wenn die Zähne der Reibe geneigt und/oder in einer Vorzugsrichtung orientiert sind. Beispielsweise können die Zähne derart angeordnet sein, dass sie in einer Bewegungsrichtung des Mahlguts bei einer Bewegung des Aufnahmeraums relativ zur Zerkleinerungsvorrichtung immer gleichbleibend orientiert sind, sich also beispielsweise die Orientierung der Zähne entlang einer Kreisbahn um die Drehachse herum ändert oder alle Zähne in die gleiche Richtung weisen.

Eine besonders effizient wirkende Zerkleinerungsvorrichtung lässt sich erhalten, wenn die Zähe durch prismenartige Vorsprünge gebildet werden, die parallel zur Drehachse durchbohrt sind. Durch Relativbewegung des Mahlguts zu den Vorsprüngen werden diese zerkleinert, insbesondere zerrieben und können aufgrund der durch Bohrungen entstehende Öffnungen durch die Zerkleinerungsvorrichtung hindurchtreten.

Eine definierte Korngröße lässt sich besonders gut dadurch vorgeben, dass die Zähne in einem quadratischen Raster angeordnet sind. Ferner kann so auch die Fertigung zur Zerkleinerungsvorrichtung optimiert und vereinfacht werden.

Um die Zerkleinerung des Mahlguts möglichst geschützt vornehmen zu können, ist es günstig, wenn ein Gehäuse vorgesehen ist und wenn das Gehäuse den Aufnahmeraum umgibt. Insbesondere kann der Aufnahmeraum derart ausgebildet sein, dass er relativ zum Gehäuse drehbar ist oder rotierbar an diesem gelagert ist.

Gemäß einer bevorzugten Ausführungsform der Erfindung kann ferner ein Mahlgutraum zum Aufnehmen des durch die Zerkleinerungsvorrichtung zerkleinerten Mahlguts vorgesehen sein. Im Mahlgutraum kann das zerkleinerte Mahlgut gesammelt werden, sodass Verluste des zerkleinerten Mahlguts minimiert oder vollständig ausgeschlossen werden können.

Die Knochenmühle lässt sich besonders einfach konstruieren und reinigen, wenn das Gehäuse mindestens einen oberen und mindestens einen unteren Gehäuseteil umfasst.

Zum Reinigen der Knochenmühle ist es von Vorteil, wenn der mindestens eine obere und der mindestens eine untere Gehäuseteil lösbar miteinander verbindbar sind.

Eine Handhabung der Knochenmühle vereinfacht sich weiter, wenn der Aufnahmeraum in mindestens einem oberen Gehäuseteil angeordnet ist.

Insbesondere dann, wenn der mindestens eine untere Gehäuseteil mit dem mindestens einen oberen Gehäuseteil lösbar verbindbar ist, ist es günstig, wenn der Mahlgutraum im mindestens einen unteren Gehäuseteil angeordnet ist. So lässt sich das zerkleinerte Mahlgut nach dem Zerkleinern auf einfache Weise aus der Knochenmühle entfernen.

Günstigerweise umfasst der mindestens eine untere Gehäuseteil die Zerkleinerungsvorrichtungsaufnahme. Insbesondere bei lösbar verbindbaren Gehäuseteilen kann so die Zerkleinerungsvorrichtung vor dem Zusammenfügen der Gehäuseteile in die Zerkleinerungsvorrichtungsaufnahme eingeführt werden. Gleichzeitig wäre es denkbar, dass die Zerkleinerungsvorrichtung den Mahlgutraum verschließt, sodass der untere Gehäuseteil vom oberen Gehäuseteil getrennt werden kann, ohne dass der Mahlgutraum vollständig freiliegt. Dadurch kann vermieden werden, dass das Mahlgut unbeabsichtigt aus dem Mahlgutraum herausfallen kann.

Vorzugsweise ist der Aufnahmeraum am oberen Gehäuseteil drehbar gelagert. Die Knochenmühle lässt sich so besonders einfach handhaben, da sie beispielsweise von einer Bedienperson ergriffen werden kann, wobei eine Bewegung des Aufnahmeraums relativ zur Zerkleinerungsvorrichtung im Inneren des Gehäuses möglich ist und gleichzeitig das Gehäuse eine Bedienperson somit auch vor Verletzungen durch die Zerkleinerungsvorrichtung schützt.

Günstig ist es, wenn ein Griffelement vorgesehen ist und wenn das Griffelement mit dem Vorschubelement verbunden oder lösbar verbindbar ist. Dadurch lässt sich zum einen das Vorschubelement auf einfache Weise relativ zur Zerkleinerungsvorrichtung bewegen, insbesondere auf diese hin, zum anderen lässt sich die Knochenmühle auch zu Reinigungszwecken besonders einfach zerlegen.

Ferner kann es vorteilhaft sein, wenn der Aufnahmeraum durch eine Bewegung des Vorschubelements in einer Richtung quer zur Zerkleinerungsrichtung relativ zur Zerkleinerungsrichtung bewegbar ist. Dies ermöglicht es, den Aufnahmeraum quasi mit dem Vorschubelement anzutreiben. Insbesondere kann eine Rotationsbewegung des Aufnahmeraums durch das Vorschubelement und eine Rotation desselben vorgegeben werden.

Günstig ist es, wenn eine Antriebseinheit vorgesehen ist zum Bewegen des Aufnahmeraums relativ zur Zerkleinerungsvorrichtung. Eine Antriebseinheit vorzusehen hat den Vorteil, dass die Knochenmühle insgesamt groß genug ausgebildet werden kann, um auch große Knochen einfach und sicher zu zerkleinern, was insbesondere mit einer handbetriebenen Knochenmühle nur schwer möglich wäre.

Grundsätzlich wäre es denkbar, eine pneumatisch oder hydraulisch betriebene Antriebseinheit zu verwenden. Vorteilhaft ist es jedoch, wenn die Antriebseinheit einen Elektromotor, einen Pneumatikmotor oder einen Hydraulikmotor umfasst. Ein Elektromotor kann beispielsweise netzabhängig oder netzunabhängig mit Strom versorgt werden. Pneumatik- und Hydraulikmotoren haben den Vorteil, dass sie gefahrlos in brandgefährdeten oder explosionsgefährdeten Umgebungen eingesetzt werden können.

Um möglichst große Kräfte auf die zu zerkleinernden Knochen übertragen zu können, ist es günstig, wenn die Antriebseinheit ein Getriebe umfasst.

Auf besonders einfache Weise lässt sich eine der oben beschriebenen Knochenmühlen mit einer Antriebseinheit ausstatten, wenn die Antriebseinheit direkt oder indirekt mit dem Vorschubelement drehfest verbunden oder verbindbar ist. Dies eröffnet insbesondere die Möglichkeit, eine zunächst für einen manuellen Betrieb vorgesehene Knochenmühle nachträglich mit einem Antrieb auszurüsten oder zu verbinden.

Gemäß einer bevorzugten Ausführungsform der Erfindung kann eine Kupplungsvorrichtung vorgesehen sein zum lösbaren Verbinden der Antriebseinheit mit dem Vorschubelement, wobei die Kupplungsvorrichtung ein erstes Kupplungselement und ein zweites, zum ersten Kupplungselement korrespondierendes Kupplungselement umfasst und wobei das erste Kupplungselement an der Antriebseinheit und das zweite Kupplungselement am Vorschubelement angeordnet oder diesem zugeordnet ist. Diese Ausgestaltung gestattet es insbesondere, die Antriebseinheit von der eigentlichen Knochenmühle zu trennen, beispielsweise zum Reinigen oder zum Beschicken der Knochenmühle mit Mahlgut. Insbesondere wäre es auch denkbar, einen im Operationssaal verfügbaren elektrischen Antrieb, beispielsweise eine chirurgische Akkumaschine, mit der Knochenmühle zu verbinden.

Um die Antriebseinheit auf einfache Weise ein- und ausschalten zu können und um optional auch eine Drehrichtung des Aufnahmeraums relativ zur Zerkleinerungsvorrichtung zu verändern, ist es günstig, wenn die Antriebseinheit mindestens ein Betätigungselement aufweist zum Betätigen der Antriebseinheit. Beispielsweise kann ein Betätigungselement in Form eines sogenannten "Gasdrückers" zum Vorgeben einer Drehzahl der Antriebseinheit vorgesehen sein, ein zweites, optionales Betätigungselement in Form eines Richtungswahlschalters, um den Aufnahmeraum im Uhrzeigersinn oder gegen den Uhrzeigersinn relativ zur Zerkleinerungsvorrichtung zu bewegen.

Damit die Knochenmühle beliebig und uneingeschränkt in einem Operationssaal eingesetzt werden kann, ist es günstig, wenn eine netzunabhängige Energieversorgungseinheit zur Energieversorgung der Antriebseinheit vorgesehen ist. Beispielsweise kann die netzunabhängige Energieversorgungseinheit eine Batterie oder eine wiederaufladbare Batterie sein.

Die nachfolgende Beschreibung bevorzugter Ausführungsformen der Erfindung dient im Zusammenhang mit der Zeichnung der näheren Erläuterung. Es zeigen:
- Fig. 1:: eine Seitenansicht einer Knochenmühle;
- Fig. 2:: eine Schnittansicht längs Linie 2-2 in Figur 1;
- Fig. 3:: eine perspektivische Darstellung eines unteren Gehäuseteils der Knochenmühle;
- Fig. 4:: eine Draufsicht auf den unteren Gehäuseteil von oben;
- Fig. 5:: eine teilweise, längs Linie 5-5 in Figur 4 geschnittene Seitenansicht des unteren Gehäuseteils;
- Fig. 6:: eine perspektivische Darstellung eines den Aufnahmeraum bildenden Elements der Knochenmühle;
- Fig. 7:: eine perspektivische Darstellung eines Vorschubelements;
- Fig. 8:: eine Draufsicht auf eine Zerkleinerungsvorrichtung;
- Fig. 9:: eine Schnittansicht längs Linie 9-9 in Figur 8;
- Fig. 10:: eine vergrößerte Ansicht des Bereichs A in Figur 8; und
- Fig. 11:: eine Schnittansicht ähnlich Figur 2 durch ein zweites Ausführungsbeispiel einer erfindungsgemäßen Knochenmühle mit einer Antriebseinheit.

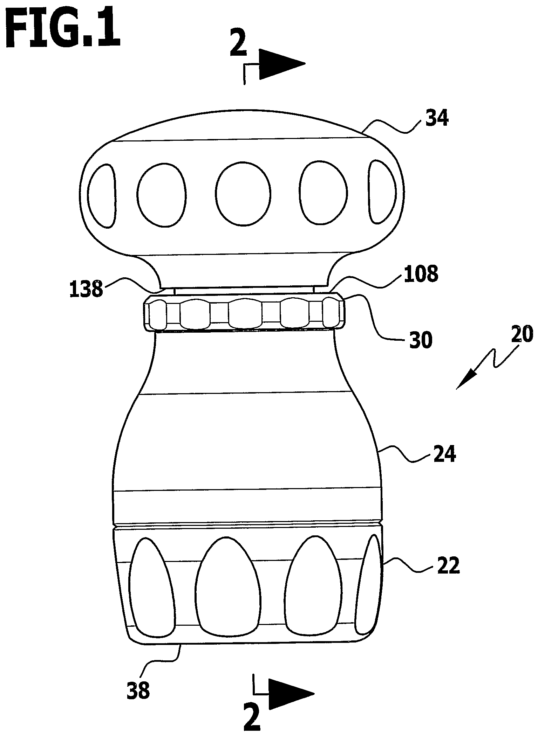

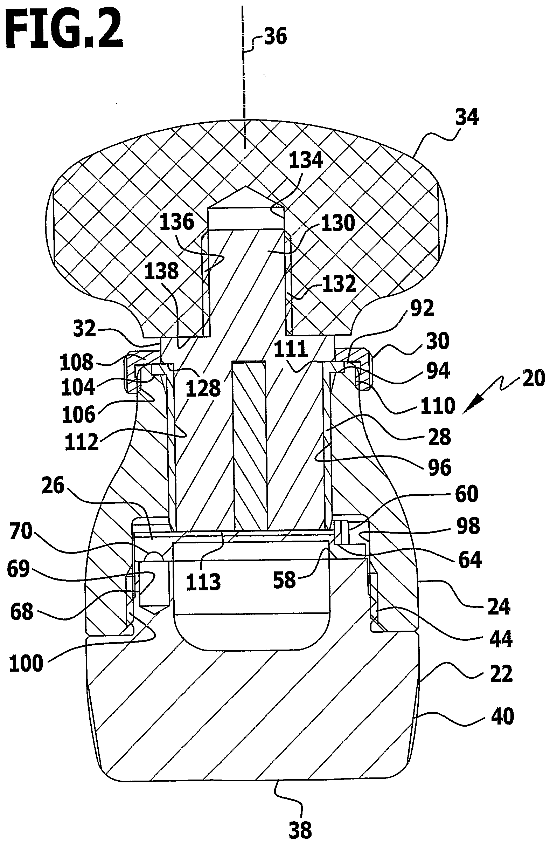

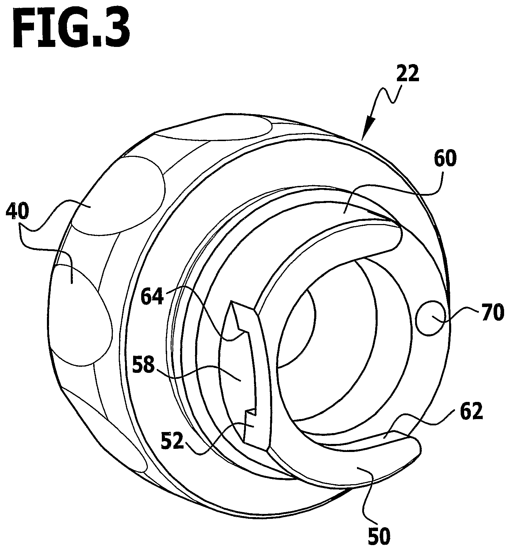

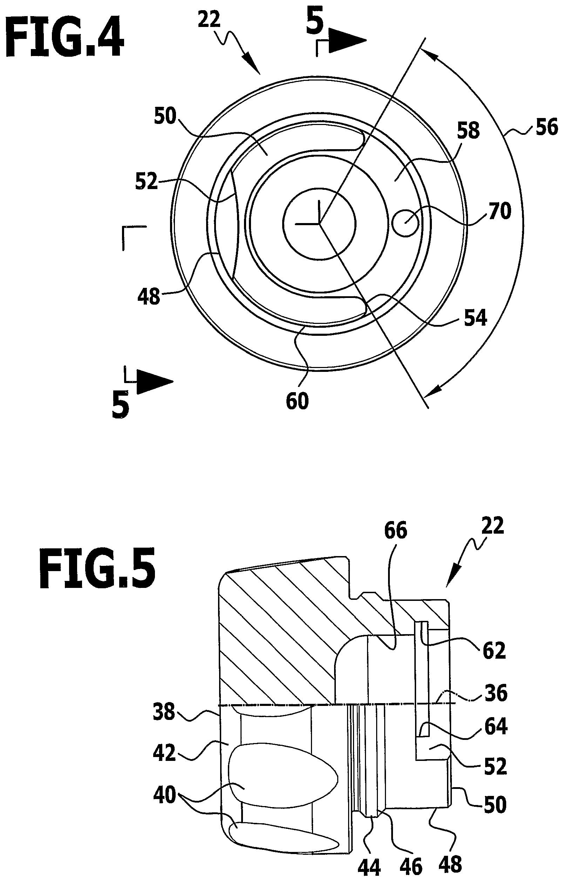

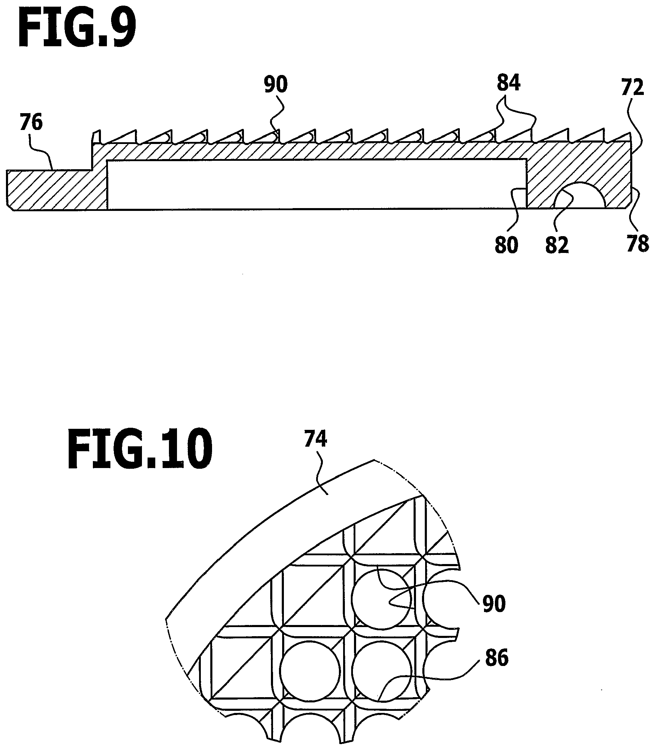

In den Figuren 1 und 2 ist eine insgesamt mit dem Bezugszeichen 20 versehene Knochenmühle dargestellt, deren einzelne Bestandteile teilweise in den Figuren 3 bis 10 dargestellt sind.

Das in den Figuren dargestellte Ausführungsbeispiel der Knochenmühle 20 umfasst sechs Bauelemente beziehungsweise Baugruppen, nämlich ein unteres Gehäuseteil 22, welches mit einem oberen Gehäuseteil 24 verschraubbar ist, eine Zerkleinerungsvorrichtung 26 in Form einer mit dem unteren Gehäuseteil 22 lösbar verbindbaren Reibe 26, einem am oberen Gehäuseteil 24 drehbar gelagerten Mahlgutbehälter 28, welcher über einen Schraubring 30 am oberen Gehäuseteil 24 gesichert ist sowie ein Vorschubelement in Form eines Stößels 32, welcher mit einem als Griffelement dienenden Knopf 34 zur einfachen Handhabung verbindbar ist.

Das untere Gehäuseteil 22 wird nachfolgend in Verbindung mit den Figuren 3 bis 5 näher erläutert.

Das untere Gehäuseteil 22 ist im wesentlichen in Form eines zu einer Längsachse 36 der Knochenmühle 20 rotationssymmetrisch geformten Körpers ausgebildet. Ein unteres Ende 38 des unteren Gehäuseteils 22 ist geschlossen und weist eine äußere schraubenartige Oberfläche 42 mit einer Vielzahl von Vertiefungen 40 zum leichteren Greifen auf. An das untere Ende 38 schließt sich oben ein mit einem Außengewinde 44 versehener Ringflansch 46 an. An den Ringflansch 46 schließt sich wiederum ein hülsenartiger Abschnitt 48 an und bildet ein oberes Ende 50 des unteren Gehäuseteils 22.

Wie in den Figuren 3 bis 5 zu erkennen, ist der Abschnitt 48 ausgehend vom Ende 50 einseitig mit einer Aussparung 52 versehen, durch welche eine Wand des Abschnitts 48 nicht vollständig, also nur teilweise, entfernt ist. Diametral gegenüberliegend ist eine größere Aussparung 54 vorgesehen, sodass eine Wand des Abschnitts 48 nur über einen Winkelbereich 56 von etwa 120° vollständig entfernt ist. Beide Aussparungen 52 und 54 reichen ausgehend vom oberen Ende 50 bis auf eine Schnittebene 58 herab.

In einem verbliebenen Wandbereich 60 ist ausgehend von der Aussparung 54 eine seitliche Nut 62 vorgesehen, die im wesentlichen parallel zu einer quer zur Längsachse 36 verlaufenden Schnittebene 58 geöffnet ist. Ferner ist eine Öffnung 64 im Wandbereich 60 durch die Aussparung 52 in Verbindung mit der Nut 62 gebildet. Die Nut 62 bildet im wesentlichen eine Zerkleinerungsvorrichtungsaufnahme, also eine Aufnahme für die Reibe 62, die parallel zur Schnittebene 58 in die Nut 62 von der Aussparung 54 her kommend eingeschoben werden kann.

Der untere Gehäuseteil ist ausgehend vom Ende 50 mit einer sacklochartigen Vertiefung 66 versehen, die einen Mahlgutraum zum Aufnehmen des durch die Reibe 26 zerkleinerten Mahlguts bildet. Im Abschnitt 48 ist im Bereich der Aussparung 54, der Aussparung 52 diametral gegenüberliegend, ein parallel zur Längsachse 32 wirkendes Kugeldruckstück 68 in einer sacklochartigen Aufnahme 69 mit einer vom Ende 50 weg federnd vorgespannten Kugel 70 angeordnet, deren Oberfläche teilweise etwas über die Schnittebene 58 hervorsteht. Wie weiter unten näher erläutert wird, bildet das Kugeldruckstück 68 ein Sicherungselement zum Sichern der Reibe 26 in der Nut 62.

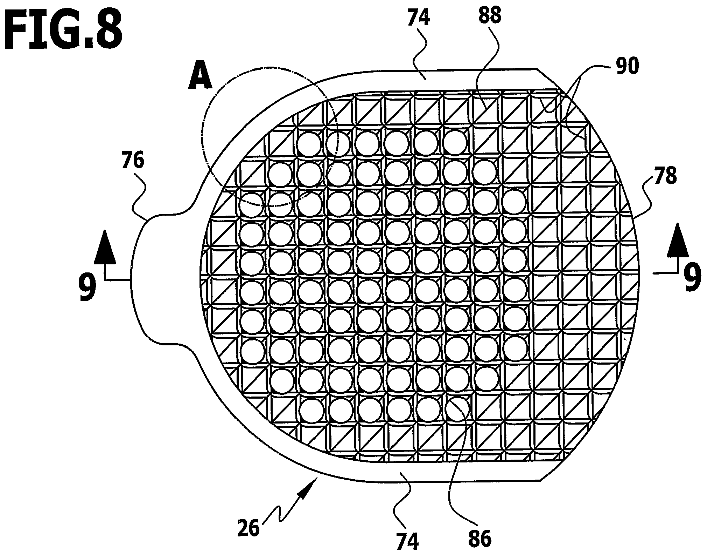

Die in den Figuren 8 bis 10 dargestellte Reibe 26 ist in Form einer Platte 72 ausgebildet, die einen äußeren Flansch 74 aufweist, welcher korrespondierend zur Nut 62 ausgebildet ist, sodass die Platte 72 von der Aussparung 54 her kommend in die Nut 62 einschiebbar ist. Am vorderen Ende der Platte ist eine kurze Zunge 76 angeformt, die durch die Öffnung 64 hindurchreicht, seitlich jedoch nicht über den Abschnitt 48 bei eingeschobener Reibe 26 hervorsteht. Ein gegenüberliegendes Ende 78 der Reibe weist einen äußeren Radius auf, der einem Radius des Abschnitts 48 entspricht.

Auf ihrer Unterseite ist die Platte 72 mit einer im Querschnitt kreisförmigen Ausnehmung 80 versehen, die bei in die Nut 62 eingeschobener Reibe 26 die Vertiefung 66 überdeckt. Ferner ist auf der Unterseite benachbart der Ausnehmung 80 eine halbkugelförmige Senkung 82 vorgesehen, die bei voll in die Nut 62 eingeschobener Reibe 26 über dem Kugeldruckstück 68 angeordnet ist, sodass die Kugel 70 in die Senkung 82 eintauchen kann.

Auf einer Oberseite der Platte 72 bilden eine Vielzahl in einem quadratischen Raster angeordnete, prismenartige Vorsprünge Zähne 84, von denen diejenigen, die vollständig über der Ausnehmung 80 angeordnet sind, jeweils von einer Bohrung 86 durchsetzt sind, deren Längsachse parallel zur Längsachse 36 orientiert ist. Die Bohrungen 86 bilden Durchtrittsöffnungen, damit durch die Zähne 84 zerkleinertes Mahlgut in den durch die Vertiefung 66 gebildeten Mahlgutraum gelangen kann.

Die prismenartigen Zähne 84 weisen alle eine quadratische Grundfläche auf und sind alle in gleicher Weise entlang einer Diagonalen 88 der Grundfläche geneigt. Zum Zerkleinern des Mahlgutes dient eine Vorderkante 90 des nach Vorsehen der Bohrungen 86 verbliebenen Teils der Zähne 84.

Mit dem unteren Gehäuseteil 22 ist das obere Gehäuseteil 24 verschraubbar. Die beiden Gehäuseteile 22 und 24 bilden einen Körper, der im Längsschnitt, wie in Figur 2 gut zu erkennen ist, im wesentlichen eine Pilzform aufweisenden Knochenmühle 20, wobei der Knopf 34 den sogenannten "Hut" des Knochenmühlen-"Pilzes" bildet. Eine Außenfläche des oberen Gehäuseteils verjüngt sich daher vom Übergang zum unteren Gehäuseteil 22 bis zu einem oberen Ende 92 desselben, welches einen Anschlag für den Schraubring 30 bildet. Am oberen Ende 92 ist eine radial nach innen weisende Aussparung 94 vorgesehen, die eine nach oben weisende ringförmige Anschlagfläche definiert. Im übrigen ist das obere Gehäuseteil 24 mit einer koaxial zur Längsachse 36 verlaufenden Durchgangsbohrung 96 versehen, die sich im Durchmesser nach etwa 3/5 einer Gesamtlänge des oberen Gehäuseteils 24 ausgehend vom Ende 92 einstufig erweitert. Im erweiterten, auf das untere Ende des oberen Gehäuseteils 24 hin weisenden Bohrungsabschnitt 98 ist ein zum Außengewinde 44 korrespondierendes Innengewinde 100 vorgesehen.

Vom Ende 92 her kommend ist in die Durchgangsbohrung 96 der Mahlgutbehälter 28 einsetzbar, welcher einen zylindrischen Grundkörper 102 aufweist, an dessen oberem Ende ein radial nach außen abstehender Ringflansch vorgesehen ist. Dieser ist so bemessen, dass er in die ringförmige Aussparung 94 eintauchen und mit seinem oberen Ende bündig mit dem Ende 92 abschließen kann. Der Mahlgutbehälter 28 ist mit dem mit einem Innengewindeabschnitt 106 versehenen Schraubring 30 sicherbar, welcher einen radial nach innen weisenden Flansch 108 trägt, der, wenn der Innengewindeabschnitt 106 auf einen korrespondierenden Außengewindeabschnitt 110 am Ende 92 des oberen Gehäuseteils 24 aufgeschraubt ist, radial nach innen den Ringflansch 104 etwas überlappt und so den Mahlgutbehälter in der Durchgangsbohrung 96 sichert.

Der Mahlgutbehälter 28 dient zur Aufnahme des zu zerkleinernden Mahlguts. Hierfür definiert er einen oben eine Einfüllöffnung und unten eine Austrittsöffnung 113 aufweisenden Aufnahmeraum 112, der durch einen hülsenförmigen Wandabschnitt 114 begrenzt wird, der sich in Umfangsrichtung in etwa über 210° erstreckt. Dieser Wandabschnitt 114 weist einen zur Längsachse 36 konzentrischen Innenwandabschnitt 116 auf. Ein von der Längsachse 36 weg weisend, konvex gekrümmter Wandabschnitt 118 ist konzentrisch zu einer parallel zur Längsachse 36 versetzten Achse 120 vorgesehen. Damit ist der konvex gekrümmte Wandabschnitt 118 exzentrisch zur Längsachse 36 angeordnet. Der konvex gekrümmte Wandabschnitt 118 ist im Querschnitt halbkreisförmig und über zwei parallel zu einer die Längsachse 36 enthaltenden Radialebene verlaufende parallele Wandabschnitte 122 mit dem Innenwandabschnitt 116 verbunden. Die Längsachse 36, die eine Symmetrieachse des Grundkörpers 102 definiert, verläuft gerade nicht durch den Aufnahmeraum 112, sondern durchsetzt einen massiven, vom konvex gekrümmten Wandabschnitt 118 begrenzten Teil des Mahlgutbehälters 28.

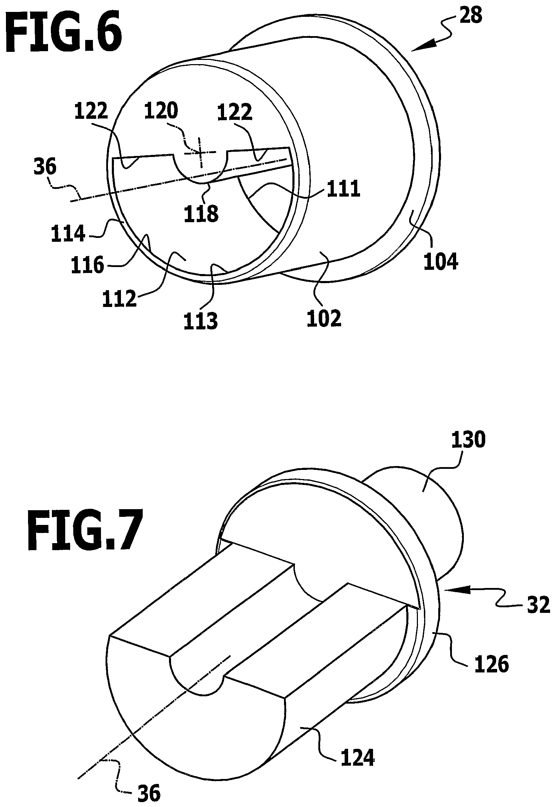

In Figur 7 ist der Stößel 32 vergrößert dargestellt, der einen Stößelkörper 124 aufweist, welcher so ausgebildet ist, dass er den Aufnahmeraum 112 formschlüssig vollständig ausfüllen kann. Außenflächen des Stößelkörpers 124 sind damit korrespondierend zu Innenflächen des Aufnahmeraums 112 ausgebildet. Der Stößelkörper 124 ist an seinem oberen Ende mit einer scheibenförmigen Platte 126 versehen, die eine nach unten weisende ringförmige Anschlagfläche 128 bildet, welche bei maximal in den Aufnahmeraum 112 vorgeschobenem Stößel 32 am Ringflansch 104 anschlägt. Ein Außendurchmesser der Platte 126 ist etwas kleiner als ein Innendurchmesser des Flansches 108 des Schraubrings 30.

Auf einer Oberseite der Platte 126 steht konzentrisch zur Längsachse 36 ein Schraubbolzen 130 ab, der mit einem Außengewinde 132 versehen ist. Der pilzhutförmige Knopf 34 weist auf seiner Unterseite eine Sacklochbohrung 134 auf, die mit einem zum Außengewinde 132 korrespondierenden Innengewinde 136 versehen ist, sodass der Knopf 34 mit dem Schraubbolzen 130 verschraubbar ist, und zwar derart, dass eine Unterseite 138 des Knopfs 34 an der Platte 126 anschlägt.

Der Zusammenbau und die Funktionsweise der Knochenmühle 20 wird nachfolgend im Zusammenhang mit den Figuren näher erläutert.

Die in ihre Bestandteile zerlegte Knochenmühle 20 kann beispielsweise durch Sterilisieren keimfrei gemacht werden. Einzig das Kugeldruckstück 68 wird dabei in der Regel nicht vom unteren Gehäuseteil 22 gelöst. Zur Vorbereitung der Knochenmühle 20 wird zunächst die Reibe 26 mit ihrem Flansch 74 in die Nut 62 eingeschoben, bis die Zunge 76 durch die Öffnung 64 hervorsteht. Die Kugel 70 rastet dann in der Senkung 82 ein. Zum Lösen der Reibe 26 vom unteren Gehäuseteil 22 kann in entgegengesetzter Richtung gegen die Zunge 76 gedrückt werden, wodurch die Kugel 70 nach unten gedrückt wird und die Senkung 82 freigibt. Die Reibe 26 kann dann aus der Nut 62 herausgezogen werden.

Der untere, mit der Reibe 26 verbundene Gehäuseteil 22 wird in einem nächsten Schritt mit dem oberen Gehäuseteil 24 verschraubt. Anschließend wird der Mahlgutbehälter 28 in die Durchgangsbohrung 96 eingesetzt und mit dem Schraubring 30 gesichert. Der Stößel 32 kann nach Verschrauben mit dem Knopf 34 in den Aufnahmeraum 112 eingeführt werden.

Zum Zerkleinern von Knochen oder Knochenmaterial wird der Stößel 32 aus dem Aufnahmeraum 112 entnommen und in diesen durch die Einfüllöffnung 111 das zu zerkleinernde Mahlgut eingefüllt. Mit dem Stößelkörper 124 wird die Einfüllöffnung 111 und damit der Aufnahmeraum 112 verschlossen und durch leichten Druck auf den Knopf 34 des Stößels 32 wird das Mahlgut gegen die Reibe 26 gedrückt. Durch Drehung des Knopfs 34 und damit des Stößels 32 wird der Mahlgutbehälter 28 und damit der Aufnahmeraum 112 um die Längsachse 36 quer zur Längsachse 36 über die Reibe 26 bewegt. Der Stößel bildet so quasi ein Antriebselement für den Mahlgutbehälter 28. Unterschreitet das zu zerkleinernde Mahlgut eine bestimmte Korngröße, kann es durch die Bohrungen 86 in die Vertiefung 66 gelangen und wird dort gesammelt. Dadurch, dass die Längsachse 36 den Aufnahmeraum 112 nicht schneidet, wird eine stets gleichbleibende Schnittgeschwindigkeit des Mahlguts relativ zur Reibe 26 bewirkt und zudem verhindert, dass zwischen dem Mahlgut und der Reibe 26 eine Nullreibung im Schnittpunkt der Längsachse 36 mit einer durch die Austrittsöffnung 113 definierten Austrittsebene entsteht. Insgesamt kann das Mahlgut nahezu rückstandsfrei und auf besonders schonende Art mit der erfindungsgemäßen Knochenmühle 20 verkleinert werden.

Vorzugsweise können alle Teile der Knochenmühle 20 aus einem sterilisierbaren Material, beispielsweise Edelstahl, hergestellt werden. Um die Handhabung der Knochenmühle 20 für eine Bedienperson noch angenehmer zu machen, kann der Knopf 34 aus einem Kunststoff hergestellt sein.

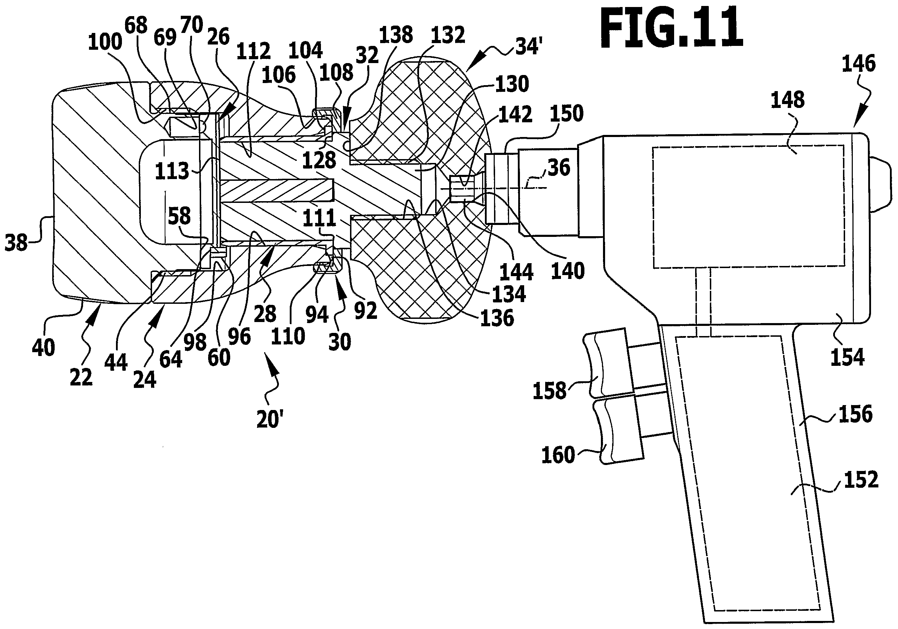

In Figur 11 ist ein zweites Ausführungsbeispiel einer erfindungsgemäßen Knochenmühle dargestellt und insgesamt mit dem Bezugszeichen 20' versehen. Die Knochenmühle 20' unterscheidet sich von der Knochenmühle 20 lediglich durch die Ausgestaltung des Knopfes 34', so dass alle übrigen Teile der Knochenmühle 20' mit denselben Bezugszeichen versehen sind wie bei der Knochenmühle 20.

Der Knopf 34' unterscheidet sich vom Knopf 34 dadurch, dass er eine Kupplungsausnehmung 140 aufweist, die vom Schraubbolzen 130 weg weisend in proximaler Richtung geöffnet ist. Vorzugsweise ist die sich in proximaler Richtung mehrstufig im Innendurchmesser erweiternde Kupplungsausnehmung 140 punktsymmetrisch zur Längsachse 36 ausgebildet. Ein Teil der Kupplungsausnehmung 140 ist vorzugsweise in Form eines Innensechskants 142 gestaltet, in den formschlüssig ein Sechskantbolzen 144 eingeführt werden kann.

Die Kupplungsausnehmung 140 dient in erster Linie der Verbindung mit einer Antriebseinheit in Form einer chirurgischen Akkumaschine 146. Diese weist eine von einem Elektromotor 148 angetriebene, nicht dargestellte Antriebswelle auf, die eine Kupplung 150, zum Beispiel in Form eines Bohrfutters, antreibt, mit der der Sechskantbolzen 144 drehfest gehalten werden kann. Als Energieversorgung für den Elektromotor 148 ist eine wiederaufladbare Batterie 152 vorgesehen, die in einen quer von einem den Elektromotor 148 umgebenden Motorgehäuse 154 schräg abstehenden Handgriff 156 eingesetzt ist. Zum Betätigen des Elektromotors 148 dienen zwei Betätigungsglieder 158 und 160, die im Wesentlichen parallel zur Längsachse 36 federnd vorgespannt und verschiebbar am Handgriff 156 gelagert sind. Das Betätigungsglied 158 dient als sogenannter "Gasdrücker", mit dem eine Drehzahl des Elektromotors 148 vorgegeben werden kann, das Betätigungsglied 160 dient als Richtungswahlschalter, wobei in Folge einer Betätigung des Betätigungsglieds 160 der Elektromotor 148 von einem Rechtslauf- in einen Linkslaufbetrieb umschaltet.

Die Akkumaschine 146 ist mit der Knochenmühle 20' lösbar verbindbar, und zwar dadurch, dass der an der Kupplung 150 gehaltene Sechskantbolzen 144 in einer Verbindungsstellung in den Innensechskant 142 am Knopf 134' formschlüssig eingreift. Optional kann zur Sicherung einer Verbindung der Akkumaschine 146 und der Knochenmühle 20' eine Sicherungsvorrichtung in Form einer Rastvorrichung vorgesehen sein, beispielsweise mit einem am Knopf 34' angeordneten, in Figur 11 nicht dargestellten Kugeldruckstück, das in der Verbindungsstellung der Akkumaschine 146 und der Knochenmühle 20' in eine entweder an der Kupplung 150 oder am Sechskantbolzen 144 ausgebildete Ausnehmung eintaucht. Alternativ wäre auch eine Schraubverbindung denkbar, um die Kupplung 150 mit dem Knopf 34' oder direkt mit den Schraubbolzen 132 drehfest zu verbinden.

Durch das Vorsehen der Akkumaschine 146 kann mit der Knochenmühle 20' weitaus härteres Knochenmaterial zerkleinert werden als mit der Knochenmühle 20, da es mit der Akkumaschine 146 möglich ist, größere Drehmomente zu erzeugen als bei einem Handbetrieb der Knochenmühle 20.

Wie bereits erwähnt, kann die chirurgische Akkumaschine 146 vorzugsweise von der Knochenmühle 20' gelöst werden, so dass die Knochenmühle 20' in analoger Weise wie die Knochenmühle 20 zerlegt und gereinigt werden kann. Eine Reinigung der Akkumaschine 146 kann separat erfolgen, vorzugsweise ist diese auch ganz oder mindestens teilweise sterilisierbar.

Alternativ zur Verbindung der Akkumaschine 146 mit dem Knopf 34' kann bei alternativen Ausführungsformen auch ganz auf den Knopf 34' verzichtet und die Kupplung 150 direkt mit dem Schraubbolzen 130 verbunden werden. Dieser kann beispielsweise anstatt des Außengewindes 132 mit einem Außensechskant versehen sein, so dass zudem auf den Sechskantbolzen 144 verzichtet werden und der mit einem Außensechskant versehene Schraubbolzen 130 direkt mit der Kupplung 150 verbunden werden kann.

## Patentansprüche

1. Knochenmühle (20; 20') zum Zerkleinern eines Mahlguts in Form von Knochen oder Knochenmaterial, mit einem Aufnahmeraum (112) für das Mahlgut, einer Zerkleinerungsvorrichtung (26) und einem Vorschubelement (32) zum Bewegen des Mahlguts im Aufnahmeraum (112) in Richtung auf die Zerkleinerungsvorrichtung (26) hin, wobei der Aufnahmeraum (112) eine Austrittsöffnung (113) aufweist, die mindestens teilweise von der Zerkleinerungsvorrichtung (26) bedeckt ist, wobei der Aufnahmeraum (112) relativ zur Zerkleinerungsvorrichtung (26) bewegbar ist und wobei der Aufnahmeraum (112) relativ zur Zerkleinerungsvorrichtung (26) um eine Drehachse (36) verdrehbar ist, **dadurch gekennzeichnet, dass** der Aufnahmeraum (112) einen bezogen auf die Drehachse (36) konvex gekrümmten Innenwandabschnitt (118) aufweist.

2. Knochenmühle nach Anspruch 1, **dadurch gekennzeichnet, dass** der Aufnahmeraum (112) eine Einfüllöffnung (111) zum Einfüllen des Mahlguts aufweist und dass die Einfüllöffnung mit dem Vorschubelement (32) teilweise oder vollständig verschließbar ist.

3. Knochenmühle nach einem der voranstehenden Ansprüche, **dadurch gekennzeichnet, dass** das Vorschubelement (32) formschlüssig in den Aufnahmeraum (112) einführbar ist.

4. Knochenmühle nach einem der voranstehenden Ansprüche, **dadurch gekennzeichnet, dass** die Zerkleinerungsvorrichtung (26) die gesamte Austrittsöffnung (113) bedeckt.

5. Knochenmühle nach einem der voranstehenden Ansprüche, **dadurch gekennzeichnet, dass** die Austrittsöffnung (113) eine Austrittsebene definiert und dass die Drehachse (36) die Austrittsebene senkrecht oder im wesentlichen senkrecht schneidet.

6. Knochenmühle nach einem der voranstehenden Ansprüche, **dadurch gekennzeichnet, dass** die Drehachse (36) die Austrittsöffnung (113) nicht schneidet.

7. Knochenmühle nach einem der voranstehenden Ansprüche, **dadurch gekennzeichnet, dass** die Drehachse (36) außerhalb des Aufnahmeraums (112) verläuft.

8. Knochenmühle nach einem der voranstehenden Ansprüche, **dadurch gekennzeichnet, dass** der Aufnahmeraum (112) einen bezogen auf die Drehachse (36) konkav gekrümmten Innenwandabschnitt (116) aufweist.

9. Knochenmühle nach Anspruch 8, **dadurch gekennzeichnet, dass** der konkav gekrümmte Innenwandabschnitt (116) konzentrisch um die Drehachse (36) herum angeordnet ist.

10. Knochenmühle nach einem der voranstehenden Ansprüche, **dadurch gekennzeichnet, dass** der konvex gekrümmte Innenwandabschnitt (118) konzentrisch oder exzentrisch um die Drehachse (36) herum angeordnet ist.

11. Knochenmühle nach einem der voranstehenden Ansprüche **dadurch gekennzeichnet, dass** der Aufnahmeraum (112) mindestens einen parallelen Wandabschnitt (122) aufweist, der parallel zu einer die Drehachse (36) enthaltenden Radialebene verläuft.

12. Knochenmühle nach Anspruch 11, **dadurch gekennzeichnet, dass** der mindestens eine parallele Wandabschnitt (122) den mindestens einen konkav gekrümmten Innenwandabschnitt (116) mit dem mindestens einen konvex gekrümmten Innenwandabschnitt (118) verbindet.

13. Knochenmühle nach Anspruch 11 oder 12, **dadurch gekennzeichnet, dass** ein Abstand des mindestens einen parallelen Wandabschnitts (122) von der Radialebene etwa dem 0,2-fachen bis 0,4-fachen eines Radius des konkav gekrümmten Innenwandabschnitts entspricht.

14. Knochenmühle nach einem der voranstehenden Ansprüche, **dadurch gekennzeichnet, dass** eine Zerkleinerungsvorrichtungsaufnahme (62) vorgesehen ist und dass die Zerkleinerungsvorrichtung (26) in die Zerkleinerungsvorrichtungsaufnahme (62) quer zur Drehachse (36) einführbar ist.

15. Knochenmühle nach Anspruch 14, **dadurch gekennzeichnet, dass** ein Sicherungselement (68) zum Sichern der Zerkleinerungsvorrichtung (26) in der Zerkleinerungsvorrichtungsaufnahme (62) vorgesehen ist.

16. Knochenmühle nach Anspruch 15, **dadurch gekennzeichnet, dass** das Sicherungselement (68) ein Kugeldruckstück ist.

17. Knochenmühle nach einem der voranstehenden Ansprüche, **dadurch gekennzeichnet, dass** die Zerkleinerungsvorrichtung (26) eine mit Zähnen (84) versehene Reibe ist.

18. Knochenmühle nach Anspruch 17, **dadurch gekennzeichnet, dass** die Zähne (84) der Reibe (26) geneigt und/oder in einer Vorzugsrichtung orientiert sind.

19. Knochenmühle nach einem der Ansprüche 17 oder 18, **dadurch gekennzeichnet, dass** die Zähne (84) durch prismenartige Vorsprünge gebildet werden, die parallel zur Drehachse (36) durchbohrt sind.

20. Knochenmühle nach einem der Ansprüche 17 bis 19, **dadurch gekennzeichnet, dass** die Zähne (84) in einem quadratischen Raster angeordnet sind.

21. Knochenmühle nach einem der voranstehenden Ansprüche, **dadurch gekennzeichnet, dass** ein Gehäuse (22, 24) vorgesehen ist und dass das Gehäuse (22, 24) den Aufnahmeraum (112) umgibt.

22. Knochenmühle nach einem der voranstehenden Ansprüche, **dadurch gekennzeichnet, dass** ein Mahlgutraum (66) vorgesehen ist zum Aufnehmen des durch die Zerkleinerungsvorrichtung (26) zerkleinerten Mahlguts.

23. Knochenmühle nach einem der Ansprüche 21 oder 22, **dadurch gekennzeichnet, dass** das Gehäuse mindestens einen oberen und mindestens einen unteren Gehäuseteil (22, 24) umfasst.

24. Knochenmühle nach Anspruch 23, **dadurch gekennzeichnet, dass** der mindestens eine obere und der mindestens eine untere Gehäuseteil (22, 24) lösbar miteinander verbindbar sind.

25. Knochenmühle nach einem der Ansprüche 23 oder 24, **dadurch gekennzeichnet, dass** der Aufnahmeraum (112) im mindestens einen oberen Gehäuseteil (24) angeordnet ist.

26. Knochenmühle nach einem der Ansprüche 23 bis 25, **dadurch gekennzeichnet, dass** der Mahlgutraum (66) im mindestens einen unteren Gehäuseteil (22) angeordnet ist.

27. Knochenmühle nach einem der Ansprüche 23 bis 26, **dadurch gekennzeichnet, dass** der mindestens eine untere Gehäuseteil (22) die Zerkleinerungsvorrichtungsaufnahme (62) umfasst.

28. Knochenmühle nach einem der Ansprüche 23 bis 27, **dadurch gekennzeichnet, dass** der Aufnahmeraum (112) am oberen Gehäuseteil (24) drehbar gelagert ist.

29. Knochenmühle nach einem der voranstehenden Ansprüche, **dadurch gekennzeichnet, dass** ein Griffelement (34; 34') vorgesehen ist und dass das Griffelement (34; 34') mit dem Vorschubelement (32) verbunden oder lösbar verbindbar ist.

30. Knochenmühle nach einem der voranstehenden Ansprüche, **dadurch gekennzeichnet, dass** der Aufnahmeraum (112) durch eine Bewegung des Vorschubelements (32) in einer Richtung quer zur Längsachse (36) relativ zur Zerkleinerungsvorrichtung (26) bewegbar ist.

31. Knochenmühle nach einem der voranstehenden Ansprüche, **dadurch gekennzeichnet, dass** eine Antriebseinheit (146) vorgesehen ist zum Bewegen des Aufnahmeraums (112) relativ zur Zerkleinerungsvorrichtung (26).

32. Knochenmühle nach Anspruch 31, **dadurch gekennzeichnet, dass** die Antriebseinheit (146) einen Elektromotor (148), einen Pneumatikmotor oder einen Hydraulikmotor umfasst.

33. Knochenmühle nach einem der Ansprüche 31 oder 32, **dadurch gekennzeichnet, dass** die Antriebseinheit (146) ein Getriebe umfasst.

34. Knochenmühle nach einem der Ansprüche 31 bis 33, **dadurch gekennzeichnet, dass** die Antriebseinheit (146) direkt oder indirekt mit dem Vorschubelement (32) drehfest verbunden oder verbindbar ist.

35. Knochenmühle nach einem der Ansprüche 31 bis 34, **dadurch gekennzeichnet, dass** eine Kupplungsvorrichtung (140, 150) vorgesehen ist zum lösbaren Verbinden der Antriebseinheit (146) mit dem Vorschubelement (32), dass die Kupplungsvorrichtung (140, 150) ein erstes Kupplungselement (144) und ein zweites, zum ersten Kupplungselement (144) korrespondierendes Kupplungselement (142) umfasst und dass das erste Kupplungselement (144) an der Antriebseinheit (146) und das zweite Kupplungselement (142) am Vorschubelement (32) angeordnet oder diesem zugeordnet ist.

36. Knochenmühle nach einem der Ansprüche 31 bis 35, **dadurch gekennzeichnet, dass** die Antriebseinheit (146) mindestens ein Betätigungselement (158, 160) aufweist zum Betätigen der Antriebseinheit (146).

37. Knochenmühle nach einem der Ansprüche 31 bis 36, **dadurch gekennzeichnet, dass** eine netzunabhängige Energieversorgungseinheit (152) zur Energieversorgung der Antriebseinheit (146) vorgesehen ist.

## Claims

1. Bone mill (20; 20') for comminuting grinding material in the form of bone or bone material, comprising a receiving chamber (112) for the grinding material, a comminuting device (26) and an advancing element (32) for moving the grinding material in the receiving chamber (112) in the direction towards the comminuting device (26), wherein the receiving chamber (112) has an exit opening (113) covered at least partially by the comminuting device (26), wherein the receiving chamber (112) is movable relative to the comminuting device (26) and wherein the receiving chamber (112) is rotatable about an axis of rotation (36) relative to the comminuting device (26), **characterized in that** the receiving chamber (112) has an inner wall section (118) curved convexly in relation to the axis of rotation (36).

2. Bone mill as defined in claim 1, **characterized in that** the receiving chamber (112) has a feed opening (111) for feeding in the grinding material and that the feed opening is adapted to be closed partially or completely by the advancing element (32).

3. Bone mill as defined in any one of the preceding claims, **characterized in that** the advancing element (32) is adapted to be inserted into the receiving chamber (112) in a form locking manner.

4. Bone mill as defined in any one of the preceding claims, **characterized in that** the comminuting device (26) covers the entire exit opening (113).

5. Bone mill as defined in any one of the preceding claims, **characterized in that** the exit opening (113) defines an exit plane and that the axis of rotation (36) intersects the exit plane at right angles or essentially at right angles.

6. Bone mill as defined in any one of the preceding claims, **characterized in that** the axis of rotation (36) does not intersect the exit opening (113).

7. Bone mill as defined in any one of the preceding claims, **characterized in that** the axis of rotation (36) extends outside the receiving chamber (112).

8. Bone mill as defined in any one of the preceding claims, **characterized in that** the receiving chamber (112) has an inner wall section (116) curved concavely in relation to the axis of rotation (36).

9. Bone mill as defined in claim 8, **characterized in that** the inner wall section (116) curved concavely is arranged concentrically around the axis of rotation (36).

10. Bone mill as defined in any one of the preceding claims, **characterized in that** the inner wall section (118) curved convexly is arranged concentrically or eccentrically around the axis of rotation (36).

11. Bone mill as defined in any one of the preceding claims, **characterized in that** the receiving chamber (112) has at least one parallel wall section (122) extending parallel to a radial plan including the axis of rotation (36).

12. Bone mill as defined in claim 11, **characterized in that** the at least one parallel wall section (122) connects the at least one inner wall section (116) curved concavely to the at least one inner wall section (118) curved convexly.

13. Bone mill as defined in claim 11 or 12, **characterized in that** a distance of the at least one parallel wall section (122) from the radial plane corresponds approximately to 0.2 times to 0.4 times a radius of the inner wall section curved concavely.

14. Bone mill as defined in any one of the preceding claims, **characterized in that** a receiving means (62) for the comminuting device is provided and that the comminuting device (26) is adapted to be inserted into the receiving means (62) for the comminuting device transversely to the axis of rotation (36).

15. Bone mill as defined in claim 14, **characterized in that** a securing element (68) for securing the comminuting device (26) in the receiving means (62) for the comminuting device is provided.

16. Bone mill as defined in claim 15, **characterized in that** the securing element (68) is a ball pressure member.

17. Bone mill as defined in any one of the preceding claims, **characterized in that** the comminuting device (26) is a grating element provided with teeth (84).

18. Bone mill as defined in claim 17, **characterized in that** the teeth (84) of the grating element (26) are inclined and/or oriented in a preferred direction.

19. Bone mill as defined in any one of claims 17 or 18, **characterized in that** the teeth (84) are formed by prism-like projections bored through parallel to the axis of rotation (36).

20. Bone mill as defined in any one of claims 17 to 19, **characterized in that** the teeth (84) are arranged in a square pattern.

21. Bone mill as defined in any one of the preceding claims, **characterized in that** a housing (22, 24) is provided and that the housing (22, 24) surrounds the receiving chamber (112).

22. Bone mill as defined in any one of the preceding claims, **characterized in that** a grinding material chamber (66) is provided for accommodating the grinding material comminuted by the comminuting device (26).

23. Bone mill as defined in any one of claims 23 or 24, **characterized in that** the housing comprises at least one upper and at least one lower housing part (22, 24).

24. Bone mill as defined in claim 23, **characterized in that** the at least one upper and the at least one lower housing part (22, 24) are releasably connectable to one another.

25. Bone mill as defined in any one of claims 23 or 24, **characterized in that** the receiving chamber (112) is arranged in the at least one upper housing part (24).

26. Bone mill as defined in any one of claims 23 to 25, **characterized in that** the grinding material chamber (66) is arranged in the at least one lower housing part (22).

27. Bone mill as defined in any one of claims 23 to 26, **characterized in that** the at least one lower housing part (22) comprises the receiving means (62) for the comminuting device.

28. Bone mill as defined in any one of claims 23 to 27, **characterized in that** the receiving chamber (112) is rotatably mounted on the upper housing part (24).

29. Bone mill as defined in any one of the preceding claims, **characterized in that** a gripping element (34; 34') is provided and that the gripping element (34; 34') is connected or releasably connectable to the advancing element (32).

30. Bone mill as defined in any one of the preceding claims, **characterized in that** the receiving chamber (112) is movable relative to the comminuting device (26) as a result of movement of the advancing element (32) in a direction transverse to the longitudinal axis (36).

31. Bone mill as defined in any one of the preceding claims, **characterized in that** a drive unit (146) is provided for moving the receiving chamber (112) relative to the comminuting device (26).

32. Bone mill as defined in claim 31, **characterized in that** the drive unit (146) comprises an electric motor (148), a pneumatic motor or a hydraulic motor.

33. Bone mill as defined in one of claims 31 or 32, **characterized in that** the drive unit (146) comprises a gear drive.

34. Bone mill as defined in any one of claims 31 to 33, **characterized in that** the drive unit (146) is non-rotatably connected or connectable directly or indirectly to the advancing element (32).

35. Bone mill as defined in any one of claims 31 to 34, **characterized in that** a coupling device (140, 150) is provided for releasably connecting the drive unit (146) to the advancing element (32), **in that** the coupling device (140, 150) comprises a first coupling element (144) and a second coupling element (142) corresponding to the first coupling element (144) and **in that** the first coupling element (144) is arranged on the drive unit (146) and the second coupling element (142) is arranged on or associated with the advancing element (32).

36. Bone mill as defined in any one of claims 31 to 35, **characterized in that** the drive unit (146) has at least one actuating element (158, 160) for actuating the drive unit (146).

37. Bone mill as defined in any one of claims 31 to 36, **characterized in that** a mains-independent energy supply unit (152) is provided for supplying energy to the drive unit (146).

## Revendications

1. Moulin à os (20; 20') destiné à broyer un produit à moudre sous la forme d'os ou d'un matériau osseux, comprenant une chambre de réception (112) pour le produit à moudre, un dispositif de broyage (26) et un élément d'avance (32) pour déplacer le produit à moudre dans la chambre de réception (112) en direction du dispositif de broyage (26), moulin à os
dans lequel la chambre de réception (112) présente une ouverture de sortie (113), qui est recouverte au moins partiellement par le dispositif de broyage (26),
dans lequel la chambre de réception (112) est mobile par rapport au dispositif de broyage (26),
et dans lequel la chambre de réception (112) peut tourner par rapport au dispositif de broyage (26), autour d'un axe de rotation (36),
**caractérisé en ce que** la chambre de réception (112) présente un secteur de paroi intérieure (118) de courbure convexe si l'on se réfère à l'axe de rotation (36).

2. Moulin à os selon la revendication 1, **caractérisé en ce que** la chambre de réception (112) présente une ouverture de remplissage (111) pour le remplissage avec le produit à moudre, et **en ce que** l'ouverture de remplissage peut être fermée partiellement ou en totalité avec l'élément d'avance (32).

3. Moulin à os selon l'une des revendications précédentes, **caractérisé en ce que** l'élément d'avance (32) peut être introduit par complémentarité de formes dans la chambre de réception (112).

4. Moulin à os selon l'une des revendications précédentes, **caractérisé en ce que** le dispositif de broyage (26) couvre la totalité de l'ouverture de sortie (113).

5. Moulin à os selon l'une des revendications précédentes, **caractérisé en ce que** l'ouverture de sortie (113) définit un plan de sortie, et **en ce que** l'axe de rotation (36) intersecte le plan de sortie de manière perpendiculaire ou sensiblement perpendiculaire.

6. Moulin à os selon l'une des revendications précédentes, **caractérisé en ce que** l'axe de rotation (36) n'intersecte pas l'ouverture de sortie (113).

7. Moulin à os selon l'une des revendications précédentes, **caractérisé en ce que** l'axe de rotation (36) s'étend à l'extérieur de la chambre de réception (112).

8. Moulin à os selon l'une des revendications précédentes, **caractérisé en ce que** la chambre de réception (112) présente un secteur de paroi intérieure (116) de courbure concave si l'on se référère à l'axe de rotation (36).

9. Moulin à os selon la revendication 8, **caractérisé en ce que** le secteur de paroi intérieure (116) de courbure concave est agencé de manière concentrique autour de l'axe de rotation (36).

10. Moulin à os selon l'une des revendications précédentes, **caractérisé en ce que** le secteur de paroi intérieure (118) de courbure convexe est agencé de manière concentrique ou excentrique autour de l'axe de rotation (36).

11. Moulin à os selon l'une des revendications précédentes, **caractérisé en ce que** la chambre de réception (112) présente au moins un secteur de paroi parallèle (122), qui s'étend parallèlement à un plan radial contenant l'axe de rotation (36).

12. Moulin à os selon la revendication 11, **caractérisé en ce que** ledit au moins un secteur de paroi parallèle (122) relie ledit au moins un secteur de paroi intérieure (116) de courbure concave au dit au moins un secteur de paroi intérieure (118) de courbure convexe.

13. Moulin à os selon la revendication 11 ou la revendication 12, **caractérisé en ce qu'**une distance dudit au moins un secteur de paroi parallèle (122) au plan radial correspond environ à 0,2 fois jusqu'à 0,4 fois un rayon du secteur de paroi intérieure de courbure concave.

14. Moulin à os selon l'une des revendications précédentes, **caractérisé en ce qu'**il est prévu un logement d'accueil de dispositif de broyage (62), et **en ce que** le dispositif de broyage (26) peut être inséré dans le logement d'accueil de dispositif de broyage (62) transversalement à l'axe de rotation (36).

15. Moulin à os selon la revendication 14, **caractérisé en ce qu'**il est prévu un élément d'arrêt de sécurité (68) pour sécuriser le dispositif de broyage (26) dans le logement d'accueil de dispositif de broyage (62).

16. Moulin à os selon la revendication 15, **caractérisé en ce que** l'élément d'arrêt de sécurité (68) est un poussoir sphérique.

17. Moulin à os selon l'une des revendications précédentes, **caractérisé en ce que** le dispositif de broyage (26) est une râpe dotée de dents (84).

18. Moulin à os selon la revendication 17, **caractérisé en ce que** les dents (84) de la râpe (26) sont inclinées et/ou orientées dans une direction préférentielle.

19. Moulin à os selon l'une des revendications 17 ou 18, **caractérisé en ce que** les dents (84) sont formées par des protubérances en forme de prisme, qui sont percées parallèlement à l'axe de rotation (36).

20. Moulin à os selon l'une des revendications 17 à 19, **caractérisé en ce que** les dents (84) sont agencées selon un quadrillage carré.

21. Moulin à os selon l'une des revendications précédentes, **caractérisé en ce qu'**il est prévu un boitier (22, 24), et **en ce que** le boitier (22, 24) entoure la chambre de réception (112).

22. Moulin à os selon l'une des revendications précédentes, **caractérisé en ce qu'**il est prévu une chambre de produit moulu (66) destinée à collecter le produit à moudre ayant été broyé par le dispositif de broyage (26).

23. Moulin à os selon l'une des revendications 21 ou 22, **caractérisé en ce que** le boitier comprend au moins une partie de boitier supérieure et au moins une partie de boitier inférieure (22, 24).

24. Moulin à os selon la revendication 23, **caractérisé en ce que** ladite au moins une partie de boitier supérieure et ladite au moins une partie de boitier inférieure (22, 24) peuvent être reliées l'une à l'autre de manière démontable.

25. Moulin à os selon l'une des revendications 23 ou 24, **caractérisé en ce que** la chambre de réception (112) est agencée dans ladite au moins une partie de boitier supérieure (24).

26. Moulin à os selon l'une des revendications 23 à 25, **caractérisé en ce que** la chambre de produit moulu (66) est agencée dans ladite au moins une partie de boitier inférieure (22).

27. Moulin à os selon l'une des revendications 23 à 26, **caractérisé en ce que** ladite au moins une partie de boitier inférieure (22) comporte le logement d'accueil de dispositif de broyage (62).

28. Moulin à os selon l'une des revendications 23 à 27, **caractérisé en ce que** la chambre de réception (112) est montée rotative sur la partie de boitier supérieure (24).

29. Moulin à os selon l'une des revendications précédentes, **caractérisé en ce qu'**il est prévu un élément de poignée (34; 34'), et **en ce que** l'élément de poignée (34; 34') est relié ou peut être relié de manière amovible à l'élément d'avance (32).

30. Moulin à os selon l'une des revendications précédentes, **caractérisé en ce que** la chambre de réception (112) peut être déplacée dans une direction transversale à l'axe longitudinal (36) par rapport au dispositif de broyage (26), par un mouvement de l'élément d'avance (32).

31. Moulin à os selon l'une des revendications précédentes, **caractérisé en ce qu'**il est prévu une unité d'entraînement (146) pour assurer le déplacement de la chambre de réception (112) par rapport au dispositif de broyage (26).

32. Moulin à os selon la revendication 31, **caractérisé en ce que** l'unité d'entraînement (146) comprend un moteur électrique (148), un moteur pneumatique ou un moteur hydraulique.

33. Moulin à os selon l'une des revendications 31 ou 32, **caractérisé en ce que** l'unité d'entraînement (146) comprend une transmission.

34. Moulin à os selon l'une des revendications 31 à 33, **caractérisé en ce que** l'unité d'entraînement (146) est reliée ou peut être reliée par une liaison fixe de rotation, directement ou indirectement à l'élément d'avance (32).

35. Moulin à os selon l'une des revendications 31 à 34, **caractérisé en ce qu'**il est prévu un dispositif d'accouplement (140, 150) pour assurer une liaison amovible de l'unité d'entraînement (146) avec l'élément d'avance (32), **en ce que** le dispositif d'accouplement (140, 150) comprend un premier élément d'accouplement (144) et un deuxième élément d'accouplement (142) correspondant au premier élément d'accouplement (144), et **en ce que** le premier élément d'accouplement (144) est agencé sur l'unité d'entraînement (146) ou est associé à celle-ci, et le deuxième élément d'accouplement (142) est agencé sur l'élément d'avance (32) ou est associé à celui-ci.

36. Moulin à os selon l'une des revendications 31 à 35, **caractérisé en ce que** l'unité d'entraînement (146) comporte au moins un élément d'actionnement (158, 160) destiné à actionner l'unité d'entraînement (146).

37. Moulin à os selon l'une des revendications 31 à 36, **caractérisé en ce qu'**il est prévu une unité d'alimentation en énergie (152) indépendante du réseau pour assurer l'alimentation en énergie de l'unité d'entraînement (146).
